# EUROPEAN PATENT SPECIFICATION

(11) **EP 2 727 595 B1**
(45) Date of publication and mention of the grant of the patent: **21.02.2018**
(21) Application number: 12805397.2
(22) Date of filing: 28.06.2012
(51) Int. Cl.: A61K 31/55, A61P 15/10, A61P 15/00, A61K 9/20

(54) **PHARMACEUTICAL COMPOSITION FOR TREATING PREMATURE EJACULATION AND METHOD FOR TREATING PREMATURE EJACULATION**
PHARMAZEUTISCHE ZUSAMMENSETZUNG ZUR BEHANDLUNG VON VORZEITIGER EJAKULATION UND VERFAHREN ZUR BEHANDLUNG VON VORZEITIGER EJAKULATION
COMPOSITION PHARMACEUTIQUE POUR TRAITER L'ÉJACULATION PRÉMATURÉE ET PROCÉDÉ POUR TRAITER L'ÉJACULATION PRÉMATURÉE

(30) Priority: 28.06.2011 KR 20110062620
(43) Date of publication of application: 07.05.2014
(60) Divisional application: 17205420.7
(73) Proprietor: CTC Bio, Inc., Seoul 138-858 (KR)
(72) Inventor: JEON, Hong-Ryeol, Suwon-si Gyeonggi-do 443-793 (KR); KWON, Do-Woo, Cheonan-si Chungcheongnam-do 331-760 (KR); LEE, Bong-Sang, Suwon-si Gyeonggi-do 443-757 (KR); KWAK, Seong-Shin, Hwaseong-si Gyeonggi-do 445-824 (KR); LEE, Sun-Ahe, Suwon-si Gyeonggi-do 443-708 (KR); PARK, Hyun-Jung, Goyang-si Gyeonggi-do 411-756 (KR); YOO, Jeong-Hwa, Hwaseong-si Gyeonggi-do 445-909 (KR)
(74) Representative: Nony
(86) International application number: PCT/KR2012/005134
(87) International publication number: WO 2013/002578

(56) References cited:
- WO-A2-02/41883
- WO-A2-2007/000764
- US-A1- 2002 037 828
- US-A1- 2003 229 001
- US-B1- 6 495 154
- ClinicalTrials NCT01439984: "Trial of PED-1 in Male Patients With Premature Ejaculation", , 22 September 2011 (2011-09-22), XP002734521, Retrieved from the Internet: URL:https://clinicaltrials.gov/archive/NCT 01439984/2011_09_22 [retrieved on 2015-01-14]
- ClinicalTrials Identifier:NCT01203202: "Phase 2 Trial of PED-1 and PED-2 in Male Patients With Premature Ejaculation", , 15 September 2010 (2010-09-15), XP002734522, Retrieved from the Internet: URL:https://clinicaltrials.gov/archive/NCT 01203202/2010_09_15 [retrieved on 2015-01-14]
- WALDINGER MARCEL D ET AL: "The majority of men with lifelong premature ejaculation prefer daily drug treatment: an observation study in a consecutive group of Dutch men.", THE JOURNAL OF SEXUAL MEDICINE JUL 2007, vol. 4, no. 4 Pt 1, July 2007 (2007-07), pages 1028-1037, XP002734523, ISSN: 1743-6095
- WALDINGER M D ET AL: "On-Demand Treatment of Premature Ejaculation with Clomipramine and Paroxetine: A Randomized, Double-Blind Fixed-Dose Study with Stopwatch Assessment", EUROPEAN UROLOGY, ELSEVIER BV, NL, vol. 46, no. 4, 1 October 2004 (2004-10-01), pages 510-516, XP004560143, ISSN: 0302-2838

## Description

### TECHNICAL FIELD

The present invention relates to a pharmaceutical composition for treating premature ejaculation, which provides rapid-onset of efficacy, exhibits less uptake deviation according to the patient's conditions and has reduced side effects. Further, the present invention relates to a medical use of the pharmaceutical composition in the treatment, prevention or improvement of premature ejaculation.

This application claims priority to Korean Patent Application No. 10-2011-0062620 filed on June 28, 2011.

### BACKGROUND ART

Premature ejaculation is one of the common sexual complaints and is estimated to affect approximately 30 to 40% of men. Premature ejaculation means persistent or recurrent ejaculation with minimal sexual stimulation before, upon, or shortly after sexual activity, and before the person wishes it. Such ejaculation that occurs sooner than desired is often disappointing and can lead to other sexual dysfunctions including erectile difficulties, female inorgasmia, low sexual desire, and sexual aversion.

It is known that premature ejaculation may be treated by using antidepressants comprising fluoxetine, paroxetine or sertraline. The Clinical Trials NCT01203202 (URL:https://clinicaltrials.gov/archive/NCT01203202/2010_09_15) discloses the phase 2 trial of clomipramine in male patients with premature ejaculation. However, the antidepressants cause side effects such as nausea, headache, dizziness, asomnia, xerostama and anxiety, and side effect associated with sedation, anticholinergic actions and cardiovascular responses. Also, considering that the antidepressants affect the nervous system, it is necessary to finely control their dosages to ensure safety. That is, unlike other drugs, since a drug for improving premature ejaculation affect the cardiovascular system and/or the nervous system which may cause severe side effects, its dosage must be carefully controlled.

Furthermore, for purposes of treating premature ejaculation, it must demonstrate a rapid onset.

### DISCLOSURE

### Technical Problem

Accordingly, it is an object of the present invention to provide a pharmaceutical composition for treating premature ejaculation, which can be conveniently taken just prior to sexual activity, not for a certain period of time, to provide rapid-onset of efficacy and exhibit outstanding effectiveness for premature ejaculation.

It is another object of the present invention to provide an optimum content, i.e. dosage of an active ingredient contained in the pharmaceutical composition.

It is yet another object of the present invention to provide a pharmaceutical composition for treating premature ejaculation, which can provide a rapid release or dissolution of an active ingredient, thereby allowing rapid uptake even if taken just prior to sexual activity and eventually exhibiting rapid-onset in its efficacy, as well as reduce an uptake deviation according to a patient and the gastrointestinal condition of the patient.

It is still another object of the present invention to provide a medical use of the pharmaceutical composition in the treatment, prevention or improvement of premature ejaculation.

### Technical Solution

In order to achieve the above objects, the present invention provides a pharmaceutical composition for treating, preventing or improving premature ejaculation, comprising clomipramine hydrochloride in an amount of 14 to 16 mg, preferably about 15mg, as an active ingredient, the composition being taken on an as-needed or on demand basis prior to sexual activity.

Also, the present invention provides clomipramine hydrochloride for use for treating, preventing or improving premature ejaculation, said clomipramine hydrochloride being administered in an amount of 14 to 16 mg to a male patient in need of the treatment of premature ejaculation. Preferably, clomipramine hydrochloride is administered to a male patient in need of the treatment of premature ejaculation, in an amount of about 15 mg based on a 70 kg male.

In addition, the present invention provides a medical use of clomipramine hydrochloride in the preparation of a medicine for treating, preventing or improving premature ejaculation, which comprises clomipramine hydrochloride in an amount of 14 to 16 mg, preferably about 15mg.

Clomipramine hydrochloride is a drug which has been long used in the treatment of depression. It is recommended that clomipramine hydrochloride is orally administered in an initial amount of 10mg/day for adults in the treatment of depression and its daily dose can gradually increase by 30 to 150 mg in divided doses for further enhanced effects. The typical daily dosage of clomipramine hydrochloride is 30 to 50 mg.

Although such a dosage of clomipramine hydrochloride is effective in the treatment of depression, it is very surprising that the use of clomipramine hydrochloride in an amount greater than about 15 mg has little to no effects in the treatment of premature ejaculation, but rather increases in side effects.

As used herein, the term "prior to sexual activity" in the directions for use of the pharmaceutical composition of the present invention, means 0.5 to 10 hours prior to sexual activity, preferably 2 to 6 hours prior to sexual activity. Considering the directions for use of the composition of the present invention taken prior to sexual activity on demand, in order to provide a rapidly-dissolved pharmaceutical composition, the composition of the present invention further comprises pregelatinized starch and sodium starch glycolate as an excipient for very rapid dissolution of clomipramine hydrochloride. More preferably, the pharmaceutical composition of the present invention comprises clomipramine hydrochloride, lactose, pregelatinized starch and sodium starch glycolate. Most preferably, the pharmaceutical composition of the present invention comprises 7 to 13 wt% of clomipramine hydrochloride, 70 to 80 wt% of lactose, 7 to 13 wt% of pregelatinized starch and 1 to 5 wt% of sodium starch glycolate based on the total weight of the composition. When the pharmaceutical composition comprising clomipramine hydrochloride according to the present invention is subject to conventional dissolution tests (using a paddle and a 900 ml dissolution medium), it exhibits a dissolution rate of 90 wt% or more, preferably 95 wt% or more at 15 minutes of testing time in all of a buffer solution of pH 1.2, a buffer solution of pH 4.0, purified water and a buffer solution of pH 6.8.

Thus, since the pharmaceutical composition comprising clomipramine hydrochloride according to the present invention comprises particular excipients in a certain amount to provide rapid drug dissolution, the efficacy of the composition taken prior to sexual activity on demand can be rapidly exhibited, and since the dissolution rate of the composition is uniform without a decrease under various pH conditions, an efficacy deviation is surprisingly reduced according to the patient's conditions.

The pharmaceutical composition of the present invention may further comprise a binder or a lubricant which are well known in the art for various purposes within the range without deteriorating the present invention. For the dissolution purpose of the present invention, povidone is preferably used as the binder.

Further, the present invention provides a medical use of the pharmaceutical composition in the treatment, prevention or improvement of premature ejaculation.

### Advantageous Effects

The present invention provides a pharmaceutical composition for treating, preventing or improving premature ejaculation, characterized by comprising clomipramine hydrochloride in an amount of 15 mg, which can provide rapid onset of efficacy, reduce a dissolution (uptake) deviation according to the patient's gastrointestinal pH conditions and minimize side effects.

Also, the present invention provides clomipramine hydrochloride for treating, preventing or improving premature ejaculation, said clomipramine hydrochloride being administered in an amount of about 15 mg based on a 70 kg male in need of the treatment of premature ejaculation.

### BEST MODE

Hereinafter, various preferred examples of the present invention will be described in detail for better understanding. The examples of the present invention are just for better understanding of the invention to persons having ordinary skill in the art.

### <Preparation of Immediate-Dissolution Formulation Comprising Clomipramine Hydrochloride >

In order to provide the desired effect immediately after intake prior to sexual activity on demand, the dissolution of a clomipramine-containing formulation should be rapidly made. Accordingly, the present inventors have endeavored to develop a formulation capable of achieving almost 100% dissolution rate within 15 minutes and found that the following prescriptions can provide very rapid dissolution and are not influenced by pH changes of a dissolution medium, thereby maintaining a high dissolution rate, through various experimentations.

Specifically, clomipramine hydrochloride-containing formulations (tablets) were prepared by the prescriptions shown in Table 1, in which povidone was used as a binder after being dissolved in purified water.

**Table 1**

| Ingredients (Amount (mg) of each ingredient contained in a tablet) | Formulation containing 15 mg of active ingredient | Formulation containing 30 mg of active ingredient |
|---|---|---|
| Clomipramine hydrochloride | 15.0 | 30.0 |
| Lactose | 111.5 | 101.1 |
| Pregelatinized starch | 14.5 | 9.5 |
| Povidone | 4.5 | 4.5 |
| Sodium starch glycolate | 3.0 | 3.0 |
| Magnesium stearate | 1.5 | 1.5 |
| Total weight | 150.0 | 150.0 |

Then, the clomipramine formulation of the present invention using the ingredients listed in Table 1 were measured for its dissolution rate according to the dissolution test method presented in the Korean Pharmacopoeia and compared with clomipramine hydrochloride-containing products which have been commercially available as antidepressants, e.g., clomipramine HCl 25 mg formulation (Myung In Pharmaceutical Co., Ltd., South Korea), clomipramine HCl 10 mg formulation (Pharmaceutical Co., Ltd., South Korea) and clomipramine HCl 25 mg formulation (Whan In Pharm. Co., Ltd., South Korea).

Dissolution rates (%) in each dissolution medium (900 ml) were represented by the proportion of clomipramine (mg) dissolved for the testing time relative to clomipramine (mg) contained in each Test Drug, and the results thereof are shown in Tables 2 to 5.

**Table 2**

| pH 1.2 | Whan In 25mg | Myung In 25mg | Myung In 10mg | Test Drug 15mg | Test Drug 30mg |
|---|---|---|---|---|---|
| 5 minutes | 51.0 | 46.7 | 38.6 | 60.6 | 53.4 |
| 10 minutes | 85.6 | 73.7 | 61.1 | 90.7 | 91.7 |
| 15 minutes | 96.6 | 90.0 | 79.7 | 99.4 | 99.5 |

**Table 3**

| pH 4.0 | Whan In 25mg | Myung In 25mg | Myung In 10mg | Test Drug 15mg | Test Drug 30mg |
|---|---|---|---|---|---|
| 5 minutes | 51.4 | 54.5 | 47.7 | 61.7 | 49.1 |
| 10 minutes | 88.5 | 91.7 | 83.3 | 94.2 | 91.8 |
| 15 minutes | 98.1 | 94.3 | 84.8 | 100.9 | 99.3 |

**Table 4**

| Purified Water | Whan In 25mg | Myung In 25mg | Myung In 10mg | Test Drug 15mg | Test Drug 30mg |
|---|---|---|---|---|---|
| 5 minutes | 48.9 | 45.0 | 54.4 | 55.4 | 47.3 |
| 10 minutes | 85.5 | 83.8 | 56.6 | 88.2 | 88.7 |
| 15 minutes | 87.7 | 88.7 | 61.6 | 99.4 | 97.8 |

**Table 5**

| pH 6.8 | Whan In 25mg | Myung In 25mg | Myung In 10mg | Test Drug 15mg | Test Drug 30mg |
|---|---|---|---|---|---|
| 5 minutes | 44.8 | 30.1 | 12.6 | 46.0 | 39.5 |
| 10 minutes | 72.3 | 54.6 | 32.2 | 95.9 | 73.9 |
| 15 minutes | 86.9 | 61.8 | 39.7 | 95.0 | 92.2 |

As shown in Tables 2 to 5, the clomipramine hydrochloride-containing formulations according to the present invention exhibited a very rapid dissolution rate and a high dissolution rate irrelevant to pH changes, as compared with other clomipramine hydrochloride-containing products available as antidepressants. These results mean that the formulations of the present invention can provide uniform efficacy irrelevant to a pH deviation in the gastrointestinal tract of the patient taking clomipramine hydrochloride.

### <Clinical Study of Rapid-Acting Clomipramine Formulation>

Male patients suffering from premature ejaculation were subject to the following parallel clinical study (Phase 2b) (randomized, double-blind, placebo-controlled and fixed dose study) for evaluating tolerance and determining a proper therapeutic capacity.

### Test Drug:

Test drugs were of 3 types, i.e., clomipramine hydrochloride 15 mg-containing formulation (PED-1) and clomipramine hydrochloride 30 mg-containing formulation (PED-2), which were prepared as shown in Table 1, and placebo with lactose as an excipient, instead of clomipramine.

### Place:

The clinical study was conducted at Seoul St. Mary's Hospital, Catholic University and Mok-dong Hospital, Ehwa Womans University.

### Purpose:

- PED-1 (clomipramine hydrochloride 15 mg), PED-2 (clomipramine hydrochloride 30 mg) and placebo were orally administered by on-demand therapy to male patients suffering from premature ejaculation , and each therapeutic capacity of PED-1 and PED-2 can be determined, relative to placebo, via the clinical study.
- Clomipramine hydrochloride 15 mg, 30mg and placebo can be compared for their effectiveness in the treatment of premature ejaculation.
- Clomipramine hydrochloride 15 mg, 30mg and placebo can be compared for their safety.

### Method:

- Subjects who passed screening entered a 4-week run-in period, and were randomized in 1:1:1 ratio of 2 groups with clomipramine and 1 group with placebo and subject to a double blind experiment. The randomized subjects showed intravaginal ejaculation latency time (IELT) of 2 minutes or less in 75% of intercourse for the run-in period. Each subject visited the hospital after taking the test drugs for 4 weeks by on-demand therapy.

### Number of Patients:

### ITT (Intent to Treatment)

- 95 patients (32 patients of placebo group, 33 patients of 15 mg group, 20 patients of 30 mg group)

### PP (Per protocol)

- 86 patients (29 patients of placebo group, 32 patients of 15 mg group, 25 patients of 30 mg group)

### Safety Population

- 92 patients (31 patients of placebo group, 33 patients of 15 mg group, 28 patients of 30 mg group)

### Body Weight and Height of Patients:

Patients had a mean body weight of 71.26±9.42 kg, and a mean height of 170.26±5.66 cm. More specifically, the placebo-administered group had a mean body weight of 71.11 kg, the 15 mg-administered group had a mean body weight of 70.85 kg, and the 30 mg-administered group had a mean body weight of 71.80 kg. All subjects were Asian.

### Patient's Conditions:

1) Patient showing IELT of 2 minutes or less in at least 75% of intercourse (on average of more than 3 times out of 4 attempts of sexual activity)
2) Male
3) Age of 20 to 65
4) Patient having a steady sexual relationship with the opposite sex of at least 6 months duration and with the intent to maintain such a relationship during the test period.
5) Male showing premature ejaculation for at least 6 months before attending the clinical study.
6) Score of more than 9 (including potential premature ejaculation) in premature ejaculation diagnostic tool (PEDT) of Korean version.
7) Healthy person having no history of a medical disorder, or any abnormalities found in a physical examination, laboratory examination, radiographic examination and electrocardiogram examination, or someone whose results departed from the reference range in a laboratory examination or electrocardiogram examination but was clearly recorded as not being clinically significant in an official document.
8) Subject whose sexual partners are women of childbearing age and agree to use contraception.
9) Patient having the will to try at least 4 attempts of sexual activity for 4 days during the baseline period without treatment.
10) Patient having the will to stop other kinds of conventional therapies (medicines, behavioral therapies or topical treatment) of premature ejaculation.

### Dosage and Method of Administration:

Group 0 was administered with a tablet of 15 mg placebo and a tablet of 30 mg placebo, Group 1 was administered with a tablet of 15 mg clomipramine HCl and a tablet of 30 mg placebo, and Group 2 was administered with a tablet of 30 mg clomipramine HCl and a tablet of 15 mg placebo for 4 weeks on-demand at about 2 hours to 6 hours prior to sexual activity. The tablets were orally taken with a glass of water.

### Evaluation of Effects:

### 1) Primary Endpoint

Primary effectiveness evaluation variables in this clinical study include an IELT fold change at visit 3 (measuring between 0 and 4 weeks) relative to baseline values (i.e., IELT fold change = Value of IELT measured at visit 3 (0 to 4 weeks) / Value of IELT measured at baseline) in each of Group 0 (with treatment of placebo), Group 1 (with treatment of 15 mg clomipramine HCl) and Group 2 (with treatment of 30 mg clomipramine HCl). Also, a difference between Group 1 and Group 0, and a difference between Group 2 and Group 0 were confirmed by a T assessment according to the Williams method.

### 2) Secondary Endpoint

① After administering placebo and 15 mg of clomipramine, and placebo and 30 mg of clomipramine for the period of 4 weeks, a change (%) of IELT values between 0 to 4 weeks after administration, relative to before administration, was calculated by the equation [Change (%) of IELT Values = (Value of IELT measured at 0 to 4 weeks - Value of IELT measured at baseline)×100 / Value of IELT measured at baseline].
② After administering placebo and 15 mg of clomipramine, and placebo and 30 mg of clomipramine for the period of 4 weeks, a mean change of IELT values between 0 to 4 weeks after administration was calculated.
③ Drug coitus interval time (DCIT) was determined by a difference between the time of dosing and the time of intercourse attempt, and the DCIT was calculated at every intercourse attempt after dosing.

### Evaluation of Safety:

### 1) Abnormal Reaction

Each group was compared for a difference in the generation of abnormal reactions according to the nature of variables through a proper statistical analysis. Also, this evaluation includes confirming the effect of a dose increase on the abnormal reactions. The abnormal reactions were summarized by encoding according to the medical dictionary for regulatory activities (MedDRA), with presenting the proportion of subjects undergoing abnormal reactions in each group and 90% confidence interval thereof. All abnormal reactions were represented according to their severity and summarized into those relevant to a test drug, those causing death or the stop of clinical trials, and those with significance. Also, if a follow-up study can be done, the results of treatment were presented. The evaluation of abnormal reactions was conducted based on abnormal reactions confirmed during the randomized treatment period.

### 2) Abnormal Change in Laboratory Examination Values

Among laboratory data obtained in this study, data which were regarded as being an abnormal value were summarized on the basis of the test group and visit order. Successive data such as blood and biochemical test results were presented by descriptive statistics based on each test group and visit order, and categorical data such as urinary test results were presented by the frequency and proportion of each category. Also, if there was a difference in test groups before and after administration of a test drug, the difference was suitably analyzed by a proper statistical method.

### 3) Abnormal Change in Physical Examination such as Electrocardiography (ECG)

Among ECG data obtained in this study, data which were regarded as being an abnormal value were summarized on the basis of the test group and visit order.

### Statistical Processing:

### 1) Primary Endpoint in Effect

Primary effectiveness evaluation variables in this clinical study include an IELT fold change relative to baseline values, which was measured before drug administration (visit 2) (the IELT fold change = IELT measured after drug administration (visit 3) / IELT measured at baseline) in each of the group with treatment of placebo, the group with treatment of 15 mg clomipramine HCl and the group with treatment of 30 mg clomipramine HCl. Also, a difference between the 15 mg clomipramine HCl group and the placebo group, and a difference between the 30 mg clomipramine HCl group and the placebo group were confirmed by the Williams test.

The results of Effective evaluation were presented by separating each of ITT and PP groups into 3 types because one subject (ID 2004) of the 15 mg clomipramine HCl group exhibited a very high value of IELT (2865.46 seconds) after 4 weeks. It was confirmed that this subject failed to ejaculate in most attempts, and thus such time was estimated as an outlier which was generated from the time to ejaculation being determined by the time until intercourse ends. Nevertheless, the time to ejaculation and the number of intercourse attempts should be recorded according to the requirements of the executed program. Owing to such an outlier, there is a high possibility that the analysis results are severely biased. For this reason, the ITT group and the PP group were each analyzed into 3 ways, i.e, 1) the analysis that the study was conducted according to the executed program (there is no change in the results of the subject of ID 2004), 2) the analysis that the value of IELT after 4 weeks corresponds to 494.690 which is the second highest score, in the 15 mg clomipramine HCl group including the subject of ID 2004, and 3) the analysis excluding the results of the subject of ID 2004.

### 2) Secondary Endpoint in Effect of Drug

Secondary effectiveness evaluation variables in this clinical study include 1) an IELT change (%) relative to baseline values, which was measured before drug administration (visit 2) (the IELT change (%) = (IELT measured after drug administration (visit 3) - IELT measured at baseline)×100 / IELT measured at baseline) in each of 3 groups, 2) a mean IELT change relative to baseline values, which was measured before drug administration (visit 2) (the mean IELT change = IELT measured after drug administration (visit 3) - IELT measured at baseline) in each of 3 groups, and 3) drug coitus interval time (DCIT) determined by a difference between the time of dosing and the time of intercourse attempt, which was presented in patient's record. In the 1) and 2) cases using IELT values, the analysis of the primary effectiveness evaluation variables was identically applied, and the 3) case using DCIT was conducted by taking account of ANOVA model so as to compare a mean value of each group.

### 3) Safety

The safety of a test drug was evaluated on the basis of all abnormal reactions, clinical laboratory results, chest X-ray results, 12-lead ECG results and vital signs (blood pressure and pulse frequency) of test subjects. All of such safety variables were obtained from baseline through randomization for the treatment period and these variables of each subject were presented and summarized by a statistical method.

For comparison of an outlier of laboratory results, chest X-ray results, 12-lead ECG results which were generated in each group, analysis was performed using chi-squared test, Fisher's exact test or Poisson test. For vital signs, the summary statistics of the successive data were presented, and assessment was performed using F-test of ANOVA.

### Results of Effect Evaluation:

The results of effect evaluation are shown in Table 6.

**Table 6**

| | | | **Clomipramine** | | |
|---|---|---|---|---|---|
| **ITT&PP*** | **Visit** | **Placebo (N=32, 29)** | **15 mg (N=33, 32)** | **30 mg (N=30, 25)** | **p-value¹⁾** |
| **ITT1** | baseline | 76.70±23.31 | 70.31±4.58 | 69.17±26.23 | 0.4968 |
| | Week 4 | 138.15±83.37 | 267.56±479.80 | 186.99±148.42 | |
| | Fold Change | 1.75±0.84 | 4.58±8.40 | 2.89±1.98 | 0.0318, 0.0030 |
| | % Change | 75.44±84.12 | 357.66±840.06 | 189.35±197.81 | 0.0318, 0.0590 |
| | change | 61.45±70.87 | 197.25±479.35 | 117.82±142.88 | 0.0030, 0.0291 |
| **ITT2** | baseline | 76.70±23.31 | 70.31±4.58 | 69.17±26.23 | 0.4968 |
| | Week 4 | 138.15±83.37 | 195.72±124.84 | 186.99±148.42 | |
| | Fold Change | 1.75±0.84 | 3.39±3.48 | 2.89±1.98 | 0.0064, 0.0030 |
| | % Change | 75.44±84.12 | 238.71±347.73 | 189.35±197.81 | 0.0064, 0.0050 |
| | change | 61.45±70.87 | 125.40±117.01 | 117.82±142.88 | 0.0030, 0.0291 |
| **ITT3** | baseline | 76.70±23.31 | 70.62±31.08 | 69.17±26.23 | 0.5125 |
| | Week 4 | 138.15±83.37 | 186.38±114.52 | 186.99±148.42 | |
| | Fold Change | 1.75±0.84 | 3.24±3.42 | 2.89±1.98 | 0.0115, 0.0030 |
| | % Change | 75.44±84.12 | 223.70±342.25 | 189.35±197.81 | 0.0115, 0.0092 |
| | change | 61.45±70.87 | 115.75±104.68 | 117.82±142.88 | 0.0030, 0.0291 |
| **PP1** | baseline | 75.66±23.98 | 70.63±31.07 | 67.92±26.13 | 0.5720 |
| | Week 4 | 137.43±87.68 | 271.19±487.01 | 203.96+155.63 | |
| | Fold Change | 1.76±0.88 | 4.64±8.53 | 3.08±1.93 | 0.0330, 0.0018 |
| | % Change | 75.91±88.02 | 364.09±852.68 | 207.85±193.30 | 0.0330, 0.0604 |
| | change | 61.77±74.40 | 200.55±486.64 | 136.05±147.95 | 0.0018, 0.0147 |
| **PP2** | baseline | 75.66±23.98 | 70.63±31.07 | 6792±26.13 | 0.5720 |
| | Week 4 | 137.43±87.68 | 197.10±126.58 | 203.96±155.63 | |
| | Fold Change | 1.76±0.88 | 3.41±3.53 | 3.08±1.93 | 0.0074, 0.0018 |
| | % Change | 75.91±88.02 | 241.42±118.72 | 207.85±193.30 | 0.0074, 0.0065 |
| | change | 61.77±74.40 | 126.47±118.72 | 136.05±147.95 | 0.0018, 0.0147 |
| **PP3** | baseline | 75.66±23.98 | 70.96±31.53 | 67.92±26.13 | 0.5818 |
| | Week 4 | 137.43±87.68 | 187.50±116.23 | 203.96±155.63 | |
| | Fold Change | 1.76±0.88 | 3.26±3.48 | 3.08±1.93 | 0.0131, 0.0018 |
| | % Change | 75.91±88.02 | 226.01±347.66 | 207.85±193.30 | 0.0131, 0.0127 |
| | change | 61.77±74.40 | 116.54±106.32 | 136.05±147.95 | 0.0018, 0.0147 |

| | | | | | |
|---|---|---|---|---|---|
| ITT(PP)1: Case defined according to the disclosure of the executed program. ITT(PP)2: Case that after the subject of ID 2004 takes a test drug, the time of intercourse applied is the maximum (494.690) of the corresponding group. ITT(PP)3: Case excluding the subject of ID 2004. 1) Comparison of Baseline IELT: F-Test (two sided); William's Test for fold change between placebo and 15 mg: one sided test with significance level 0.0250; William's Test for fold change between placebo and 30 mg: one sided test with significance level 0.0250; William's Test for change and % change between placebo and 15 mg: one sided test with significance level 0.0250; William's Test for change and % change between placebo and 30 mg: one sided test with significance level 0.0250. | | | | | |

As a result of this clinical study for premature ejaculation patients who ejaculated within 2 minutes after vaginal insertion, all dosing group (15 mg, 30mg) of clomipramine exhibited the extended time of ejaculation after vaginal insertion. In particular, when a degree of extended ejaculation time after vaginal insertion is represented by the value of IELT fold change after the treatment period of 4 weeks relative to before a test drug-treatment (baseline), the analysis of ITT groups showed that the value of IELT fold change was estimated to 4.58±8.40, 3.39±3.48, and 3.24±3.42 in the clomipramine 15 mg group according to the treatment result of each outlier, while 1.75±0.84 in the placebo group, and also the value of IELT fold change in the clomipramine 30 mg group was 2.89±1.98. In the case of ITT1, it was confirmed that there is no significant difference (p=0.0318) between the placebo group and the clomipramine 15 mg group at one sided test with a significant level of 2.5%. However, in the cases of ITT2 and ITT 3, it was confirmed that there is a notable increase (p=0.0064, 0.0115) in the value of IELT fold change of the clomipramine 15 mg group, as compared with that of the placebo group. Also, comparing the clomipramine 30 mg group with the placebo group, there is a notable difference (p=0.0030) at one sided test with a significant level of 2.5%. In conclusion, the minimum effective dose in the case of ITT1 was 30 mg of clomipramine, while each minimum effective dose in the cases of ITT2 and ITT3 was 15 mg of clomipramine. When considering overall situations including these results and the fact that an outlier fails to reflect the nature of disorders well, 15 mg of clomipramine can be determined as the minimum effective dose, and it would be understood that there is no difference between 15 mg of clomipramine and 30 mg of clomipramine in terms of IELT fold change being an evaluation base of the primary effectiveness.

### Results of Safety Evaluation:

The results of safety (regarding abnormal reactions) evaluation are shown in Tables 7 to 9.

**Table 7**

| | | **Clomipramine** | | |
|---|---|---|---|---|
| | **Placebo** | **15 mg** | **30 mg** | **Total** |
| | **(N=34)** | **(N=34)** | **(N=33)** | **(N=101)** |
| Number of Subjects showing Abnormal Reactions | 4(11.76) | 11(32.35) | 19(57.57) | 34(34.00) |
| | | | | |
| Generation Number of Abnormal Reactions | 4 | 19 | 30 | 53(53%) |
| Mild | 4 | 17 | 28 | 49 |
| Moderate | 0 | 2 | 2 | 4 |
| Severe | 0 | 0 | 0 | 0 |
| Number of Subjects showing Serious Abnormal Reactions | 0 | 0 | 0 | 0 |
| Generation Number of Serious Abnormal Reactions | 0 | 0 | 0 | 0 |

**Table 8**

| | | **Clomipramine** | | |
|---|---|---|---|---|
| **System Organ Class** | **Placebo** | **15 mg** | **30 mg** | **Total** |
| | **(N=33)** | **(N=34)** | **(N=33)** | **(N=100)** |
| Gastrointestinal disorders | 1 | 6 | 8 | 15 |
| General disorders | | 2 | 4 | 6 |
| Infections | | | 1 | 1 |
| Investigations | 1 | 1 | 2 | 4 |
| Nervous system disorders | | 4 | 4 | 8 |
| Psychiatric disorders | | 3 | 8 | 11 |
| Renal and urinary disorders | | 2 | 1 | 3 |
| Reproductive system and breast disorders | 1 | | 1 | 2 |
| Skin and subcutaneous tissue disorders | 1 | 1 | 1 | 3 |

**Table 9**

| | | **Clomipramine** | | |
|---|---|---|---|---|
| **System Organ Class** | **Placebo** | **15 mg** | **30 mg** | **Total** |
| | **(N=34)** | **(N=34)** | **(N=33)** | **(N=100)** |
| Number of Subjects Showing Abnormal Reactions | 3(8.82) | 10(29.41) | 17(51.51) | 30(30.00) |
| Number of Abnormal Reactions having Causal Relationship | 3 | 15 | 26 | 44 |
| Follow-up | | | | |
| No | 3 | 12 | 16 | 31 |
| Readministration After Temporary Suspension | | | 1 | 1 |
| Continuous Stop of Medication | | 3 | 9 | 12 |
| Progressing | | | | |
| Complete Disappearance | 3 | 15 | 26 | 44 |

In the safety evaluation of subjects who were administered with a test drug or placebo after randomization, most of the subjects showed mild abnormal reactions, without serious or severe abnormal reactions. Although the abnormal reactions were mild, the generation rate of abnormal reactions, which were estimated to have a causal relationship with a test drug, and the proportion of the corresponding subjects were shown to be dose-dependent.

### Conclusion:

In conclusion, taking into consideration all the results of ITT groups and PP groups into account, since the 15mg dose groups exhibited an effect of extension in intravaginal ejaculation latency time (IELT) after insertion, as compared with the placebo groups, and also exhibited good drug tolerance, it is believed that a dose of 15mg would be suitable as a treatment dose.

## Claims

1. A pharmaceutical composition for use for treating, preventing or improving premature ejaculation, comprising clomipramine hydrochloride in an amount of 14 to 16 mg as an active ingredient, the composition being taken on demand prior to sexual activity, wherein the composition further comprises pregelatinized starch and sodium starch glycolate, and wherein the composition exhibits a dissolution rate of 90 wt% or more at 15 minutes of testing time in all of a buffer solution of pH 1.2, a buffer solution of pH 4.0, purified water and a buffer solution of pH 6.8, when the composition is subject to a dissolution test using a paddle and a 900 ml dissolution medium

2. The pharmaceutical composition for use of claim 1, which comprises clomipramine hydrochloride in an amount of 15 mg.

3. The pharmaceutical composition for use of claim 1, which is administered 2 to 6 hours prior to sexual activity.

4. The pharmaceutical composition for use of any one of claims 1 to 3, which comprises clomipramine hydrochloride, lactose, pregelatinized starch and sodium starch glycolate.

5. The pharmaceutical composition for use of claim 4, which comprises 7 to 13 wt% of clomipramine hydrochloride, 70 to 80 wt% of lactose, 7 to 13 wt% of pregelatinized starch and 1 to 5 wt% of sodium starch glycolate based on the total weight of the composition.

6. The pharmaceutical composition for use of claim 5, which further comprises a binder or a lubricant.

7. The pharmaceutical composition for use of claim 6, wherein the binder is povidone.

8. Clomipramine hydrochloride for use in the treatment, prevention and improvement of premature ejaculation, said clomipramine hydrochloride being administered to a male patient 2 to 6 hours prior to sexual activity in an amount of 14 to 16 mg, wherein the clomipramine hydrochloride is administered in a pharmaceutical composition further comprising pregelatinized starch and sodium starch glycolate.

9. Clomipramine hydrochloride for use according to claim 8, wherein said Clomipramine hydrochloride is administered in an amount of 15 mg.

## Patentansprüche

1. Pharmazeutische Zusammensetzung zur Verwendung bei der Behandlung, Prävention oder Verbesserung von vorzeitiger Ejakulation, umfassend Clomipramin-Hydrochlorid in einer Menge von 14 bis 16 mg als Wirkstoff, wobei die Zusammensetzung bei Bedarf vor sexueller Aktivität eingenommen wird, wobei die Zusammensetzung ferner vorverkleisterte Stärke und Natriumstärkeglykolat umfasst und wobei die Zusammensetzung eine Auflösungsrate von 90 Gew.-% oder mehr bei einer Testzeit von 15 Minuten in einer Pufferlösung mit einem pH-Wert von 1,2, einer Pufferlösung mit einem pH-Wert von 4,0, gereinigtem Wasser und einer Pufferlösung mit einem pH-Wert von 6,8 aufweist, wenn die Zusammensetzung einem Auflösungstest unter Verwendung eines Rührblatts und eines 900-ml-Auflösungsmediums unterworfen wird.

2. Pharmazeutische Zusammensetzung zur Verwendung nach Anspruch 1, die Clomipramin-Hydrochlorid in einer Menge von 15 mg umfasst.

3. Pharmazeutische Zusammensetzung zur Verwendung nach Anspruch 1, die 2 bis 6 Stunden vor sexueller Aktivität verabreicht wird.

4. Pharmazeutische Zusammensetzung zur Verwendung nach einem der Ansprüche 1 bis 3, die Clomipramin-Hydrochlorid, Lactose, vorverkleisterte Stärke und Natriumstärkeglykolat umfasst.

5. Pharmazeutische Zusammensetzung zur Verwendung nach Anspruch 4, die 7 bis 13 Gew.-% Clomipramin-Hydrochlorid, 70 bis 80 Gew.-% Lactose, 7 bis 13 Gew.-% vorverkleisterte Stärke und 1 bis 5 Gew.-% Natriumstärkeglykolat, bezogen auf das Gesamtgewicht der Zusammensetzung, umfasst.

6. Pharmazeutische Zusammensetzung zur Verwendung nach Anspruch 5, die ferner ein Bindemittel oder ein Schmiermittel umfasst.

7. Pharmazeutische Zusammensetzung zur Verwendung nach Anspruch 6, wobei es sich bei dem Bindemittel um Povidon handelt.

8. Clomipramin-Hydrochlorid zur Verwendung bei der Behandlung, Prävention oder Verbesserung von vorzeitiger Ejakulation, wobei das Clomipramin-Hydrochlorid einem Patienten 2 bis 6 Stunden vor sexueller Aktivität in einer Menge von 14 bis 16 mg verabreicht wird, wobei das Clomipramin-Hydrochlorid in einer pharmazeutischen Zusammensetzung verabreicht wird, die ferner vorverkleisterte Stärke und Natriumstärkeglykolat umfasst.

9. Clomipramin-Hydrochlorid zur Verwendung nach Anspruch 8, wobei das Clomipramin-Hydrochlorid in einer Menge von 15 mg verabreicht wird.

## Revendications

1. Composition pharmaceutique pour utilisation pour le traitement, la prévention ou l'amélioration de l'éjaculation précoce, comprenant du chlorhydrate de clomipramine en une quantité de 14 à 16 mg en tant que substance active, la composition étant prise à la demande avant l'activité sexuelle, la composition comprenant en outre de l'amidon prégélatinisé et du glycolate d'amidon sodique, et la composition présentant un taux de dissolution de 90 % en poids ou plus à 15 minutes de temps d'essai dans toutes parmi une solution tampon à pH 1,2, une solution tampon à pH 4,0, de l'eau purifiée et une solution tampon à pH 6,8, lorsque la composition est soumise à un essai de dissolution en utilisant un appareil à palettes et 900 ml de milieu de dissolution.

2. Composition pharmaceutique pour utilisation de la revendication 1, qui comprend du chlorhydrate de clomipramine en une quantité de 15 mg.

3. Composition pharmaceutique pour utilisation de la revendication 1, qui est administré 2 à 6 heures avant une activité sexuelle.

4. Composition pharmaceutique pour utilisation de l'une quelconque des revendications 1 à 3, qui comprend du chlorhydrate de clomipramine, du lactose, de l'amidon prégélatinisé et du glycolate d'amidon sodique.

5. Composition pharmaceutique pour utilisation de la revendication 4, qui comprend 7 à 13 % en poids de chlorhydrate de clomipramine, 70 à 80 % en poids de lactose, 7 à 13 % en poids d'amidon prégélatinisé et 1 à 5 % en poids de glycolate d'amidon sodique sur la base du poids total de la composition.

6. Composition pharmaceutique pour utilisation de la revendication 5, qui comprend en outre un liant ou un lubrifiant.

7. Composition pharmaceutique pour utilisation de la revendication 6, dans laquelle le liant est la povidone.

8. Chlorhydrate de clomipramine pour utilisation dans le traitement, la prévention et l'amélioration de l'éjaculation précoce, ledit chlorhydrate de clomipramine étant administré à un patient de sexe masculin 2 à 6 heures avant une activité sexuelle en une quantité de 14 à 16 mg, le chlorhydrate de clomipramine étant administré dans une composition pharmaceutique comprenant en outre de l'amidon prégélatinisé et du glycolate d'amidon sodique.

9. Chlorhydrate de clomipramine pour utilisation selon la revendication 8, ledit chlorhydrate de clomipramine étant administré en une quantité de 15 mg.
